(19) 

Europäisches
Patentamt
European
Patent Office
Office européen
des brevets

(11) **EP 2 571 574 B1**

(12) **EUROPEAN PATENT SPECIFICATION**

(45) Date of publication and mention
of the grant of the patent:
**08.04.2015 Bulletin 2015/15**

(21) Application number: **11735701.2**

(22) Date of filing: **05.05.2011**

(51) Int Cl.:
***A61N 7/00*** *(2006.01)*

(86) International application number:
**PCT/IL2011/000360**

(87) International publication number:
**WO 2011/138784 (10.11.2011 Gazette 2011/45)**

(54) **SYSTEM OF OPERATING A MULTI FOCUSED ACOUSTIC WAVE SOURCE**

SYSTEM DES BETRIEBS EINER SCHALLWELLENQUELLE MIT MEHREREN FOKUSSEN

SYSTÈME D'UTILISATION DE SOURCE D'ONDES ACOUSTIQUES À PLUSIEURS FOYERS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.07.2010 US 364471 P
05.05.2010 US 331451 P**

(43) Date of publication of application:
**27.03.2013 Bulletin 2013/13**

(73) Proprietors:
- **Technion Research & Development Foundation
Ltd.
32000 Haifa (IL)**
- **Yissum Research Development Company of The
Hebrew
University of Jerusalem
91390 Jerusalem (IL)**

(72) Inventors:
- **SHOHAM, Shy
32985 Haifa (IL)**
- **KIMMEL, Eitan
47212 Ramat-HaSharon (IL)**
- **NAOR, Omer
36584 Doar-Na HaAmakim (IL)**

(74) Representative: **Gulde & Partner
Patent- und Rechtsanwaltskanzlei mbB
Wallstraße 58/59
10179 Berlin (DE)**

(56) References cited:
**US-A1- 2010 030 076**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD AND BACKGROUND OF THE INVENTION

The present invention, relates to system of operating an acoustic wave source and, more particularly, but not exclusively, to using acoustic energy of an acoustic wave source for diagnosis, stimulation and/or inhibition.

Focused acoustic waves (or shockwaves, the terms being used interchangeably throughout) are being used increasingly in medical applications. For example, acoustic waves are used for tissue ablation, diagnostic imaging, drug delivery, breaking up concretions in the body such as kidney stones, treating orthopedic diseases, combating soft tissue complaints and pain, and other therapies which employ heat, cavitation, shock waves, and other thermal and/or mechanical effects for therapeutic purposes.

Typically electrical energy is converted into acoustic waves, such as by generating a strong pulse of an electric or magnetic field, usually by capacitor discharge, converting the electromagnetic field into acoustic energy, and directing the energy to a target by means of an associated focusing apparatus.

A neural interface is a device which allows recording and interpreting the behavior of neurons, or changing their behavior. For example, a brain-computer interface (BCI), sometimes called a direct neural interface or a brain-machine interface, is a direct communication pathway between a brain and an external device. Neural activity is based on neurons undergoing rapid depolarization and eliciting Action Potentials (spikes) which are the elements of the neural code. Affecting neural activity may take the form of stimulation whereby neurons are made to spike on demand, inhibition whereby spiking is completely blocked, or gentler modulation of the probability of spiking.

Some methods of modulating neural activity are performed by applying US acoustic energy. For example, see Fry, F.J., H.W. Ades, and W.J. Fry, Production of Reversible Changes in the Central Nervous System by Ultrasound. Science, 1958. 127(3289): p. 83-84et al., and Gavrilov, L.R., E.M. Tsirulnikov, and I.a.I. Davies, Application of focused ultrasound for the stimulation of neural structures. Ultrasound in medicine & biology, 1996. 22(2): p. 179-192, which are incorporated herein by reference. In a recent study, described in Tyler, W.J., et al., Remote Excitation of Neuronal Circuits Using Low-Intensity, Low-Frequency Ultrasound. PLoS ONE, 2008. 3(10): p. e3511, which is incorporated herein by reference, it has been shown that ultrasonic energy may be used for stimulating neural structures within the mammalian Central Nervous System (CNS). Further, ultrasound pulses caused ion flux transients through ion channels in the membranes of neurons in the hippocampal tissue and depolarization of membrane potential leading to action potentials and transmitter release from pre-synaptic terminals. Other studies showed stimulation of the motor cortex and hippocampus of mice *in-vivo,* for example Yusuf Tufail, Alexei Matyushov, Nathan Baldwin, Monica L. Tauchmann, Joseph Georges, Anna Yoshihiro, Stephen I. Helms Tillery, William J. Tyler, Transcranial Pulsed Ultrasound Stimulates Intact Brain Circuits, Neuron, 2010; 66 (5): 681-694, which is incorporated herein by reference, and stimulation of motor cortex and suppression of activity in the visual cortex of rabbits *in-vivo,* Yoo, S.-S., A. Bystritsky, J.-H. Lee, Y. Zhang, K. Fischer, B.-K. Min, N. J. McDannold, A. Pascual-Leone and F. A. Jolesz (2011), "Focused ultrasound modulates region-specific brain activity", Neuro Image In Press, Corrected Proof, which is incorporated herein by reference.

A mechanical model of the interaction between ultrasound waves and biological membranes offers an explanation for the mechanism behind ultrasound-based modulation of neural activity, see Krasovitski, B., V. Frenkel, S. Shoham and E. Kimmel, 2011, "Intramembrane cavitation as a unifying mechanism for ultrasound-induced bioeffects", Proceedings of the National Academy of Sciences, in press.

SUMMARY OF THE INVENTION

According to the present disclosure there is provided a method of operating a multi focused acoustic wave source. The method comprises providing the multi focused acoustic wave source, providing a plurality of target acoustic pressures to be applied on a plurality of regions of interest (ROIs) in at least one cellular tissue, computing a transmission pattern of multi-focal acoustic energy according to the plurality of target acoustic pressures, and operating the multi focused acoustic wave source according to the transmission pattern.

Optionally, the at least one cellular tissue is a retina of the eye.

Optionally, each the target acoustic pressure is different from another the target acoustic pressures.

Optionally, the providing comprises providing a spatiotemporal pattern for applying the plurality of target acoustic pressures each vary over a period in a different the ROI.

More optionally, the period is a predefined period.

Optionally, the providing comprises receiving instructions for applying the plurality of target acoustic pressures, each in a different the ROI; wherein the instructions are generated according to readings of at least one sensor.

More optionally, the at least one sensor is selected from a group consisting of: a video camera, an image sensor, a pressure sensor, a pressure transducer, a proximity sensor, and an acoustic to electric sensor.

Optionally, the method further comprises analyzing a functional response of the at least one cellular tissue to the target acoustic pressures.

Optionally, the computing comprises computing a transmission spatiotemporal pattern defining a plurality of phases each for another of a plurality of dynamic acoustic energy elements, the operating being performed by adjusting the plurality of dynamic acoustic energy elements to transmit according to the plurality of phases.

More optionally, each phase is weighted according to a relative location of a respective the dynamic acoustic energy element.

Optionally, the plurality of target acoustic pressures is neural interface signal, the at least one cellular tissue comprising a neural tissue.

Optionally, the computing comprises computing a plurality of phases for a plurality of acoustic energy transmissions, the operating comprising operating the multi focused acoustic wave source to transmit the plurality of acoustic energy transmissions with the plurality of phases.

More optionally, the amplitudes of the plurality of acoustic energy transmissions are substantially similar.

More optionally, the plurality of phases are computed according to a random superposition (SR) process.

More optionally, the plurality of phases are computed according to a Gerchberg-Saxton (GS) process.

More optionally, the plurality of phases are computed according to a weighted Gerchberg-Saxton (GSW) process.

More optionally, the plurality of phases are computed according to a pseudo-inverse (PINV) process.

Optionally, each the ROI is a three dimensional space.

Optionally, the providing comprises providing a desired bioeffect and selecting the plurality of target acoustic pressures according to the desired bioeffect.

Optionally, the computing comprises computing a transmission spatiotemporal pattern defining a plurality of amplitudes each for another of a plurality of dynamic acoustic energy elements, the operating being performed by adjusting the plurality of dynamic acoustic energy elements to transmit according to the plurality of amplitudes.

Optionally, the method further comprises computing a speckle reduction adjustment for the transmission pattern, the operating being performed according to the speckle reduction adjustment.

According to some embodiments of the present invention there is provided a system of patterning a multi-focal acoustic energy transmission. The system comprises an input interface which receives a plurality of target acoustic pressures to be applied on at a plurality of interest (ROIs) in at least one cellular tissue, a computing unit which computes a transmission pattern of multi-focal acoustic energy according to the plurality of target acoustic pressures, and a controller which operates a source of multi-focal acoustic energy to transmit multi-focal acoustic energy field according to the transmission pattern.

Optionally, the computing unit computes a speckle reduction adjustment for the transmission pattern, the controller operates the source according to the transmission pattern in light of the speckle reduction adjustment.

Optionally, the source having a plurality of dynamic acoustic energy elements, the transmission pattern is a spatiotemporal pattern defining a plurality of excitation phases each for another of the plurality of dynamic acoustic energy elements.

More optionally, the system further comprises a measuring unit which measures a reaction of the at least one cellular tissue to the multi-focal acoustic energy field.

More optionally, the system further comprises a man machine interface for allowing a user to select the plurality of target acoustic pressures.

According to the present disclosure there is provided a method of operating a multi focused acoustic wave source. The method comprises providing an arrangement of a plurality of dynamic acoustic energy elements, providing a plurality of target acoustic pressures to be applied on a plurality of regions of interest (ROIs) in at least one cellular tissue, patterning a transmission of multi-focal acoustic energy from the plurality of dynamic acoustic energy elements according to the plurality of target acoustic pressures, and outputting the pattern.

Optionally, the patterning comprises patterning a plurality of phases of the multi-focal acoustic energy according to the plurality of target acoustic pressures.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting. The invention itself is defined in the claims.

Implementation of the system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volitile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

## BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

FIG. 1 is a schematic illustration of a system of patterning a multi-focal acoustic energy, optionally ultrasound, according to some embodiments of the present invention;

FIG. 2A is a schematic illustration of an exemplary architecture of the system depicted in FIG. 1, according some embodiments of the present invention;

FIG. 2B is a schematic illustration of an exemplary ultrasonic phased array probe, according some embodiments of the present invention;

FIG. 2C is a schematic illustration of an exemplary ultrasonic phased array probe that is installed on a lens of glasses, according some embodiments of the present invention;

FIG. 3 is a flowchart of a method of operating a multi focused acoustic wave source, for diagnosing, stimulating, and/or inhibiting neural activity, according to some embodiments of the present invention;

FIGs. 4A and 4B depict a phase map of a transmission pattern for producing a single focus in a certain ROI and the resultant acoustic intensity map, respectively;

FIGs. 5A and 5B depict a phase map of a transmission pattern calculated using GSW algorithm for nine foci, according to some embodiments of the present invention, and the resultant acoustic intensity map, respectively;

FIG. 6A is an exemplary ultrasonic intensity map which is generated by calculations which are based on PINV and GSW algorithms, in arbitrary units of intensity, for a 987 element phased array, according to some embodiments of the present invention;

FIG. 6B is a graph depicting the focal tightness of a single generated focus and a multiple foci on a 9-foci grid, on the horizontal and vertical axes, according to some embodiments of the present invention;

FIG. 6C includes a set of graphs that compare mean efficiencies and uniformities measured for sets of 12 pseudorandom and symmetric maps generated by the four differ calculations, according to some embodiments of the present invention;

FIG. 7A depicts a thermal image that maps the temperature elevation generated after 13.2 seconds of sonication induced by the pattern calculated by the GSW algorithm, according to some embodiments of the present invention;

FIG. 7B is a graph depicting the relationship between the intensity and the transverse distance from the center of the phased-array, according to some embodiments of the present invention;

FIG. 7C is a thermal image that maps the temperature elevation induced by the pattern used to created the image in FIG. 7A, where the generation is based on compensating weights, according to some embodiments of the present invention;

FIG. 7D is a graph depicting focal tightness as quantified for a single central focus and a graph depicting the compensated temperature elevation maps after 13.2 seconds of sonication induced by the pattern calculated by the GSW algorithm, as quantified for a single central focus, according to some embodiments of the present invention;

FIG. 7E depicts a complex pattern created by 22 focal points where the temperature elevation was measured 19.8 seconds after onset of a sonication induced by the pattern calculated by the GSW algorithm, according to some embodiments of the present invention;

FIG. 8 depicts simulations performed using 2048x2048 pixels matrixes of complex numbers to span an acoustic field plane of 10.2x10.2cm at 50x50$\mu$m resolution where three letter patterns, 'A', 'B' and 'C', were manually built into 1.5x1.5cm masks (the row in marked with the notation A);

FIG. 9 depicts averaged traces from one experiment session that shows evident responses to flash and ultrasound (ultrasound, uncoupled ultrasound, and ultrasound + injection of tetrodotoxin) stimuli; and

FIG. 10 is a graph depicting grouped, averaged and normalized results of the response to flash and US stimulations.

DESCRIPTION OF EMBODIMENTS OF THE INVENTION

The present invention, in some embodiments thereof, relates to method and system of operating a multi focused acoustic wave source and, more particularly, but not exclusively, to using acoustic energy of a multi focused acoustic wave source for diagnosis, stimulation and/or inhibition.

According to some embodiments of the preset invention there is provided a system of operating a multi focused acoustic wave source to apply a plurality of target acoustic pressures on a plurality of regions of interest in one or more cellular tissues, for brevity referred to herein as a cellular tissue, according to a certain transmission pattern, optionally a spatiotemporal pattern which changes over time in one or more dimensions. The certain transmission pattern is set according to computer generated holography (CGH) algorithms, for example random mask algorithms, random superposition algorithms, and/or Gerchberg-Saxton GS algorithms and/or Weighted Gerchberg-Saxton GS algorithms or other algorithms. When the wave source has multiple elements which are constrained to have very similar or even identical amplitudes, the transmission spatiotemporal pattern given by these algorithms is substantially optimal with respect to different aspects of the required target pressure field, such as total delivered power or uniformity of power delivery to different targets. Other types of algorithms may be used to define the transmission pattern, for example pseudo inverse algorithms.

According to some embodiments, the target acoustic pressures has a plurality of ROIs, which may be referred to herein alternatively as ROIs or target sites, which may be referred to as foci when the acoustic pressure is applied, for example 3, 9, 25, 36, 64, 128, and/or any intermediate or larger number. In such an embodiment, different two dimensional and/or three dimensional patterns, optionally spatiotemporal patterns, of, pressures may be applied on a plurality of regions of interest of different tissues, such as neural tissues, and/or for inducing different bioeffects. Optionally, the acoustic pressure at different foci may be similar, identical and/or different. Optionally, the pressure at different foci may change and/or remain constant over time.

The method is based on a multi focused acoustic wave source that optionally has a plurality of dynamic acoustic energy elements, such as ultrasound transducers. First, target acoustic pressures to be applied on a plurality of region of interests (ROIs) in a cellular tissue, referred to herein as an ROI, is provided. Then, a transmission pattern, optionally spatiotemporal, of multi-focal acoustic energy is computed according to the target acoustic pressures. As used herein, a dynamic transmission pattern means a transmission pattern which varies in time, either according to predefined instructions and/or in real time, according to the readings of one or more sensors. This allows operating the multi focused acoustic wave source according to the transmission pattern.

The system, which may be used as a neuro-stimulation device, includes an input interface, such as a man machine interface, which receives target acoustic pressures to be applied on a plurality of ROIs in a cellular tissue. The target acoustic pressures may be selected according to one or more desired bioeffects. Optionally, the user inputs or selects one or more desired bioeffects and the target acoustic pressures which are adapted to induce the desired bioeffect is automatically selected. The system further includes a computing unit which computes a transmission pattern of multi-focal acoustic energy according to the target acoustic pressures and a controller which operates a source of multi-focal acoustic energy to transmit multi-focal acoustic energy according to the transmission pattern.

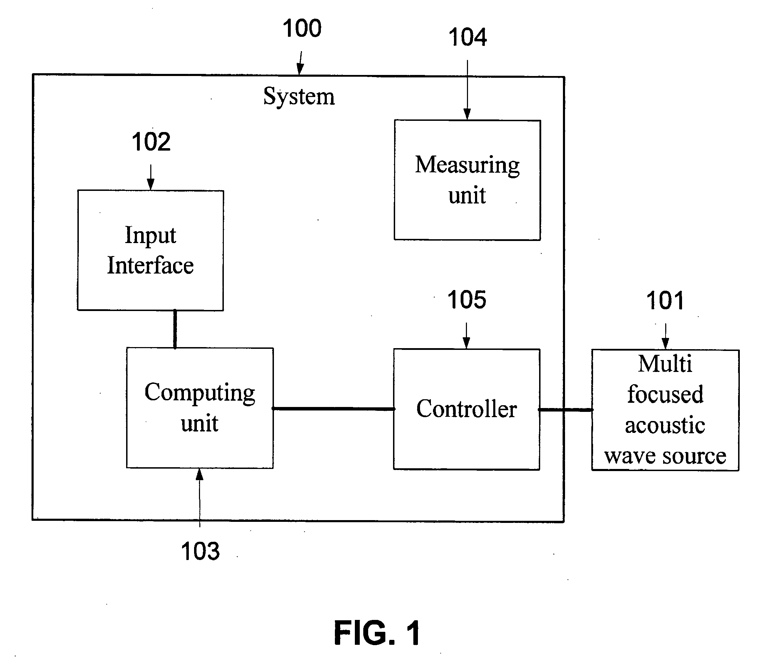

Reference is now made to FIG. 1, wherein is a schematic illustration of a system 100 of patterning a multi-focal acoustic energy, optionally ultrasound, transmission, according to some embodiments of the present invention. Optionally, the system 100 is a neuro-stimulation device where the patterning allows applying different target acoustic pressures on a neural tissue, for example as a neural interface.

The system 100 is set to operate a multi focused acoustic wave source, such as a probe that includes an array of ultrasound transducers each optionally separately controllable to be activated independently in a different fashion. The multi focused acoustic wave source may include an ultrasound source, such as a focused ultrasound (FUS) source, a high-Intensity focused ultrasound (HIFU) source and/or a magnetic resonance-guided focused Ultrasound (MRgFUS) source. This probe may be referred to herein as an ultrasonic phased array probe 101. The multi focused multi focused acoustic wave source 101 generates a tight, intense and electronically steerable focal region from a distributed source, optionally in fields with multiple simultaneous foci or spatially extended focal regions. The multi focused acoustic wave source controls both the phase and the amplitude of a generated wavefront.

Optionally, the system 100 includes an input interface 102 which provides a plurality of target acoustic pressures for applying on a plurality of target sites for brevity referred to herein as regions of interest (ROIs), which may be referred to herein as points or target points, in a target area or a target space. The target acoustic pressures may be selected according to a desired bioeffect, for example neural stimulation, and/or neural inhibition or a desired diagnosis. The ROI may be a target organ or a part of a target organ, such as the brain, the eye and optionally the retina there within, and/or any other neural system or network. The stimulation and/or inhibition may be used for restoring and/or creating a composite sensory perception for people who have a major deficiency in their sensory systems, such as blindness or deafness with varying degrees. Employing the multi-focal capabilities to stimulate or otherwise modulate the activity in primary sensory cortices or in the retina and auditory cochlea if these are functioning, would allow a stream of sensory input from the outside world to the brain, considering spatial and/or temporal resolutions.

According to some embodiments of the present invention, the input interface 102 receives instructions from a controller that continuously translates sensory input from an acquisition unit, such as an image sensor, a pressure sensor, pressure transducer and/or proximity sensor, an acoustic to electric sensor, such as one or more microphones and the like. This translation allows using the system 100 for generating desired stimulation patterns, which optionally dynamically change, in real time according to the controller's instructions. In such embodiments patterned acousto-stimulation may be formed for any of the following or to any combination thereof:

A. Restoration or creation of a composite sensory perception for individuals who have a major deficiency in their sensory systems
B. Motor restoration in paralyzed individuals through spinal cord patterned acousto-stimulation.
C. Diagnostic pre-operative or intra-operative procedures where the patterned acousto-stimulation is used for functional assessment, for example, detection of epileptic foci.
D. Systems for treating obesity, depression, chronic pain and other nervous system disorders based on patterned acousto-stimulation.
E. Systems where a disordered stimulation pattern or an inhibitory or modulatory pattern disables an errant neural pattern, including obsessive-compulsive disorder.
F. Systems where multiple interacting brain regions are activated simultaneously.

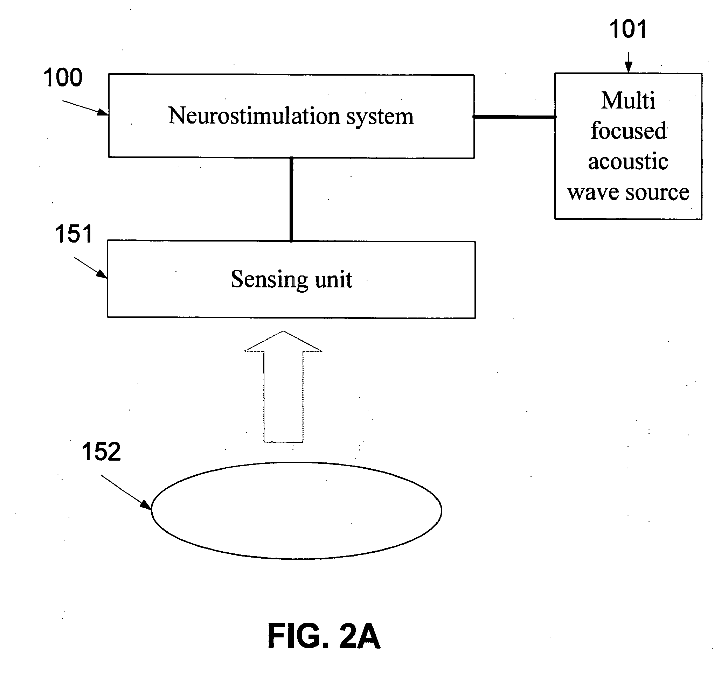

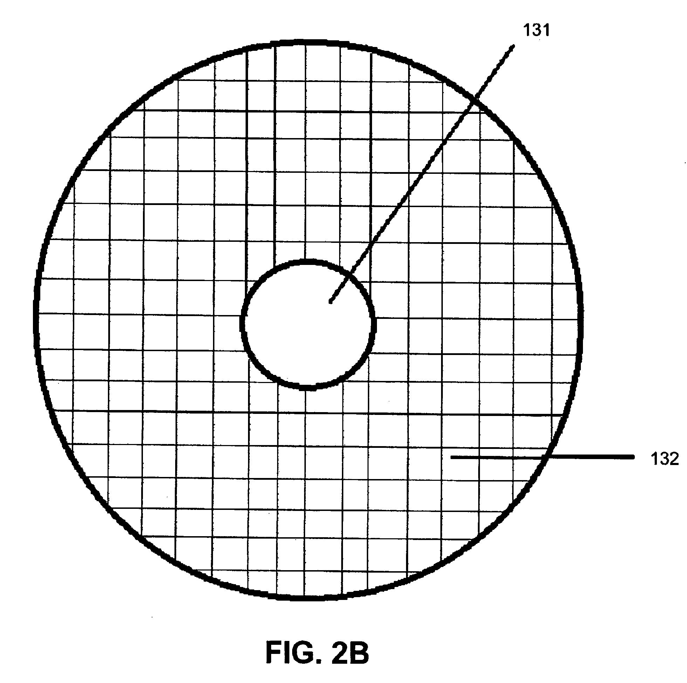

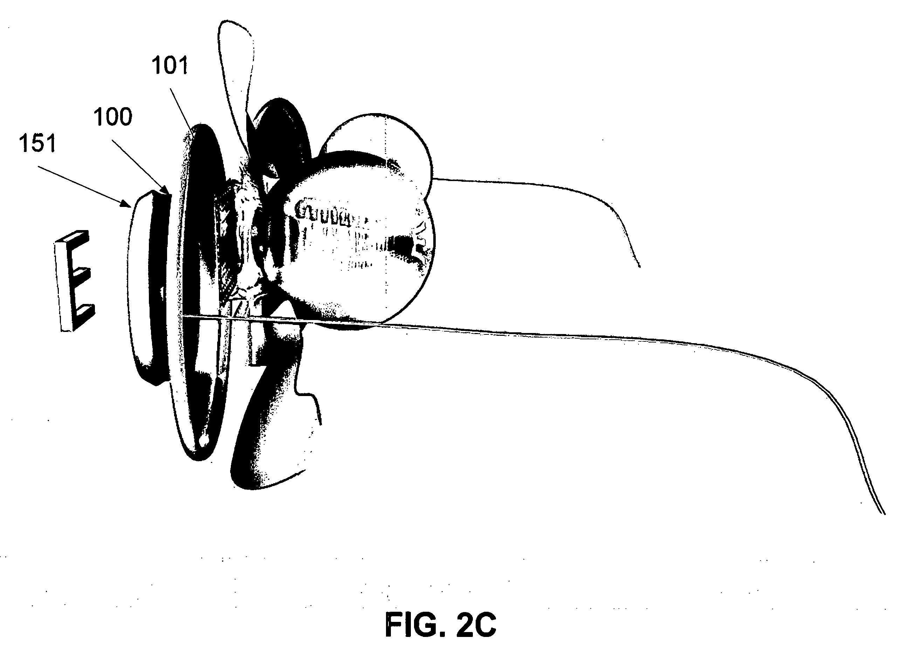

According to some embodiments of the present invention, the system is a neuroprosthesis system 100 which is connected to a sensing unit 151, for example as depicted in FIG. 2. In such an embodiment, the System 100 operates as described above. The Sensing unit 151 serves for sensing information from the environment 152 and transmitting signals pertaining to the sensed information to input interface 102 of the neuroprosthesis system 100. The neuroprosthesis system 100 calculates a stimulation pattern (e.g., by means of a data processor as further detailed hereinabove) based on the information and operates the multi focused acoustic wave source 101 to encode the stimulation pattern to a target location. The sensing unit 151 may be embodied in many forms. In some embodiments of the present invention sensing unit 151 collects visual information. For example, the sensing unit may be an imaging device which captures an image of a scene and transmits it to neurostimulation system 100. In these embodiments, the stimulation pattern corresponds to visual information and the target area is the retina or the visual cortex. In some embodiments of the present invention neurostimulation system 100 collects acoustical information. For example, sensing unit 151 may include a microphone which collects acoustic waves from the environment and converts them to electrical signals and a transmitter which transmits the signals to the neurostimulation system 100. In these embodiments, the stimulation pattern corresponds to the acoustic information and the target area is the cochlea or the auditory cortex. Other types of sensing units are not excluded from the scope of the present invention.

System 100 or part thereof can be mounted on the subject by any known technique. For example, when system 100 is used to stimulate neurons in the retina, sensing unit 151 may be mounted on a head-up display as known in the art. Alternatively, sensing unit 151 may be miniaturized and implanted in the eye. When system 100 is used to stimulate neurons in the cochlea, sensing unit 151 can be miniaturized and mounted in or behind the ear and/or miniaturized and implanted in the cochlea.

Neural inhibition effects may be achieved, for example, by transmitting energy, either continuously or not, for periods from about 10 milliseconds to 15 minutes, in frequencies ranging for example from about 0.25 MHz, for example 1 MHz to about 20 MHz and pulse temporal acoustic intensities, $I_{PA}$, ranging up to 100W/cm^2 for example from 1-80 $^{W}/_{cm}{}^2$, see, inter alia, Tyler, W.J., et al., Remote Excitation of Neuronal Circuits Using Low-Intensity, Low-Frequency Ultrasound. PLoS ONE, 2008. 3(10): p. e3511 and Colucci, V., et al., Focused Ultrasound Effects on Nerve Action Potential in vitro. Ultrasound in Medicine & Biology, 2009. 35(10): p. 1737-1747, which are incorporated herein by reference. Neural stimulation may be achieved, for example, with transmission periods of about 20 $\mu$sec to about 5 sec, in frequencies ranging for example from about 0.25, for example 1 MHz to about 10 MHz, for example 5 MHz and $I_{PA}$ ranging for example from about 20 $^{mW}/_{cm}{}^2$ - 100 $^{W}/_{cm}{}^2$ see Yusuf Tufail, Alexei Matyushov, Nathan Baldwin, Monica L. Tauchmann, Joseph Georges, Anna Yoshihiro, Stephen I. Helms Tillery, William J. Tyler. Transcranial Pulsed Ultrasound Stimulates Intact Brain Circuits. Neuron, 2010; 66 (5): 681-694, Yoo, S.-S., A. Bystritsky, J.-H. Lee, Y. Zhang, K. Fischer, B.-K. Min, N. J. McDannold, A. Pascual-Leone and F. A. Jolesz (2011). "Focused ultrasound modulates region-specific brain activity." NeuroImage In Press, Corrected Proof and Muratore, R., J. LaManna, E. Szulman, M.S.A. Kalisz, M. Lamprecht, M.S.M. Simon, M.S.Z. Yu, N. Xu, and B. Morrison. Bioeffective Ultrasound at Very Low Doses: Reversible Manipulation of Neuronal Cell Morphology and Function in Vitro, in 8TH INTERNATIONAL SYMPOSIUM ON THERAPEUTIC ULTRASOUND, 2009. Optionally, the input interface 102 includes a user interface (UI) that allows an operator and/or an imaging processing module to select a target area that confines the ROIs. For example, the target organ may be displayed on a screen, allowing a user to encircle the ROIs using a man machine interface (MMI), such as a keyboard, a touch screen and a mouse.

In the above, the pulse average intensity ($I_{PA}$) means the intensity computed using this formula:

$$I_{PA} = \frac{1}{PD} \int_{t_0}^{t_0+PD} \frac{p^2(t)}{Z_0} dt \,,$$ in which PD is the duration of a US pulse, $t_0$ is the time at which a pulse begins, $p(t)$ is the instantaneous pressure and $Z_0$ is the characteristic acoustic impedance of the sonicated material.

The system 100 includes a computing unit 103, such as a central processor and a memory, which computes a focused acoustic energy transmission to be applied on the ROIs according to the target acoustic pressures. The computing unit 103 optionally computes a transmission pattern which patterns the transmission of multi-focal acoustic energy according to the received target acoustic pressures.

For example, the computing unit 103 computes the excitation phases required for each acoustic energy element, for example transducer, in the array in order to achieve a desired modulation in the required coordinates, for example as described below. In use, the computing unit 103 instructs a multi focused acoustic wave source controller 105 to the multi focused acoustic wave source 101 according to the computed transmission pattern. The transmission pattern is calculated according to one or more generated holography (CGH) algorithms, for example as further described below.

Optionally, the system includes or is connected to a measuring unit 104, which measures the reaction of the cellular tissue, optionally a neural tissue, in the ROIs to the focused acoustic energy transmission, for example the excitation level. This may be performed by direct measurement device, for example a functional magnetic resonance imaging (fMRI) module and/or an electroencephalogram (EEG) and/or indirect measurement device, such as electromyography (EMG) module that measures signals from excited muscles. The data from the measuring unit may be used as a feedback interface. The feedback may be manually or semi-automatically utilized by a user and/or a medical device, or optionally to be used as input to an automated feedback controller. The feedback may be used to alter and/or improve the employed transmission pattern to achieve more desirable effects.

In use, the multi focused multi focused acoustic wave source 101 creates an ultrasonic field that has an effect on the neural tissue in the ROIs. The ultrasonic field may be calculated using a Fourier transform in case the desired modulation is in a two dimensional (2D) plane, or using a Fresnel transform in the case the desired modulation is in a three dimensional (3D) space, or some modification of these transforms, as for example is shown below.

The variability in amplitudes between the transducers may be limited by a demand such as equal average power consumption and/or even constrained to be uniform. Thus, the task of a computing unit 103 may be either to compute an inverse Fourier or Fresnel transform, or a modified version of such transforms, if arbitrary amplitudes are allowed, or an optimal phase-only solution if not. Optionally, inverse transforms may be computed while the amplitudes of all transducers set to be substantially similar, for example identical, and a random phase is added to each transducer, for example according to a random superposition (SR) equation solution. Optionally, a phase-only SR solution is calculated and then the field the SR solution creates is repeatedly computed by a forward transform, retaining the phases while computing array phases which are required for a phase-only field, by an inverse transform, until the error between a desired field and a resulting field is lower than a threshold. Optionally, the computing is performed by calculating extensions of the GS algorithms, such as the weighted GS (GSW) which results in better uniformity, see Di Leonardo R, Ianni F and Ruocco G 2007 Computer generation of optimal holograms for optical trap arrays Opt. Exp. 15 1913-22. The specific algorithm to use is a matter to be considered when designing a device for a specific application, considering the available hardware, importance of high efficiency and uniformity and the trade-off between generating a more accurate field and obtaining a rapid solution.

In some neuro-degenerative diseases of the retina such as Retinitis Pigmentosa (RP) or Age-related Macular Degeneration (AMD), photoreceptor cells in the neural tissue are damaged and not functioning properly while other neural cells, for example bi-polar cells, horizontal cells, amacrine cells, ganglion cells, etc., are still functioning to some extent. While natural sight depends on the proper function of photoreceptors, external activation of the other cell types by multifocal ultrasound may allow restoration of sight to a certain degree. However, in some cases of such afflictions, some photoreceptor cells still function properly, so that a person afflicted with such a disease may still have some residual vision, meaning that some limited ability to see remains.

For the purpose of modulating the activity of a retina of such a diseased person, it may be advantageous to use the system 100 with a specific design for the ultrasonic phased array. For example, as depicted in FIG. 2B, such an ultrasonic phased array 132 may be designed to have a hole 131 and/or a region of transparency, through which light may pass with little or no attenuation.

In this way, at the same time that the device produces multi-focal ultrasonic patterns for stimulating the retina, light also falls on the retina, activating the photoreceptor cells in the tissue which are still responsive to it.

Optionally, in such a system the ultrasonic phased array probe 101 is attached to the eye, so that its position in space with respect to the eye's pupil is constant. If the ultrasonic phased array probe 101 is designed to have a hole and / or a transparent region, as depicted in FIG. 2B, then the hole, or region of transparency, admits light's entrance through the pupil, disregarding eye movements. More optionally, the ultrasonic phased array probe 101 is attached to the eye using a hydrogel, such as the hydrogels used in contact lenses. Optionally this may be a silicone hydrogel, such as used for silicone hydrogel contact lenses. More optionally, the ultrasonic phased array probe 101 is placed on lens of glasses, as depicted in FIG. 2C.

Optionally, the ultrasonic phased array probe 101 has a planar geometry, meaning that the composing acoustic elements reside on about the same plane.

Optionally, the ultrasonic phased array probe 101 has a curved geometry, for example a spherical segment, or for example an ellipsoidal segment, such that the acoustic elements reside on a surface which is a part of a sphere, or a part of an elliposoid.

Different materials may be used to create the ultrasonic phased array probe 101. Optionally, the ultrasonic phased array probe 101 is created from a piezo-electric material, for example a piezo-ceramic material such as Lead Titanate (PT), Bismuth Titanate, Barium Titanate, Lead Metaniobate (PMN), Lithium Niobate and Lead Zirconate Titanate (PZT). More optionally, the ultrasonic phased array probe 101 is a piezo-polymer device, using for example the polymer polyvinylidene fluoride (PVDF) or its co-polymer with tri-fluoroethylene (P[VDF-TrFE]), or a polyvinyl chloride polymer or co-polymers of Nylon.

As an alternative option, the ultrasonic phased array probe 101 is based on silicone or silicone compounds. For example, it may be manufactured using capacitive micro-machined ultrasonic transducer (CMUT) technology, such as described in S. H. Wong, M. Kupnik, R. D. Watkins, K. Butts-Pauly, and B. T. Khuri-Yakub ("Capacitive micromachined ultrasonic transducers for therapeutic ultrasound applications" ,IEEE Tran. Biomed. Eng., vol. 57, no. 1, pp. 114-123, Jan. 2010) or in K. K. Park, H. Lee, M. Kupnik, and B. T. Khuri-Yakub, ("Fabrication of capacitive micromachined ultrasonic transducers via local oxidation and direct wafer bonding" Microelectromechanical Systems, Journal of, vol. 20, no. 2, pp. 95-103, Feb. 2011).

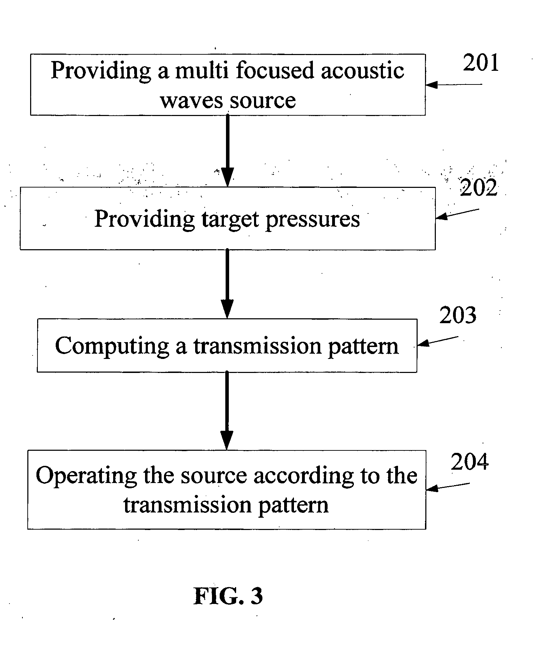

Reference is now made to FIG. 3, which is a flowchart of a method of operating a multi focused acoustic wave source, such as shown at 101, for diagnosing, stimulating, and/or inhibiting neural activity, according to some embodiments of the present invention.

First, as shown at 201, a multi focused acoustic wave source having a plurality of dynamic acoustic energy elements, such as N transducers, is provided.

As shown at 202, a plurality of target acoustic pressures each in a different ROI, are provided, for example selected or defined by the user and/or image analysis software. As used herein, target acoustic pressures define different target acoustic pressures which are applied in a plurality of target sites. The target acoustic pressures may be applied on ROIs which have one, two, or three dimensions, defining one or more acoustic energy transmission foci on one or more target sites. The target acoustic pressure in each site may be constant or temporal, namely changing over a certain period. Each target acoustic pressure may be manually defined, for example using a graphical user interface (GUI) that is presented to the user and/or automatically according to an automatic analysis of an image, optionally volumetric, of the ROIs, for example in a target tissue, for example a computerized tomography (CT) image, an magnetic resonance imaging (MRI) image, a positron emission tomography (PET)-CT image, a single photon emission computed tomography (SPECT) image and the like. The pressure applied in each one of the target points may be uniform or non uniform.

According to some embodiments, the target acoustic pressures are applied in a plurality of target sites, which may be referred to as foci when the acoustic pressure is applied, for example 2, 3, 9, 25, 36, 64, 128, and/or any intermediate or larger number. In such an embodiment, different two dimensional and/or three dimensional patterns of pressures may be applied on different tissues, such as neural tissues, and/or for inducing different bioeffects. Optionally, the acoustic pressure at different foci may be similar, identical and/or different. Optionally, the pressure at different foci may change and/or remain constant over time.

Now, as shown at 203, an acoustic energy transmission pattern is computed according to the target acoustic pressures. As used herein, an acoustic energy transmission pattern means a set of instructions for operating the multi focused acoustic wave source to generate one or more acoustic energy transmissions, optionally multifocal, using the plurality of acoustic energy elements. The acoustic energy transmission pattern optionally defines the characteristics of each acoustic energy transmission of each one of the plurality of acoustic energy elements during one or more transmission cycles. The characteristics optionally include the phase and optionally the amplitude and/or frequency phase of the transmission. Optionally, the transmission pattern is selected according to a desired amount of pressure to be applied on one or more target neurons. The acoustic energy elements may be operated sequentially and/or simultaneously.

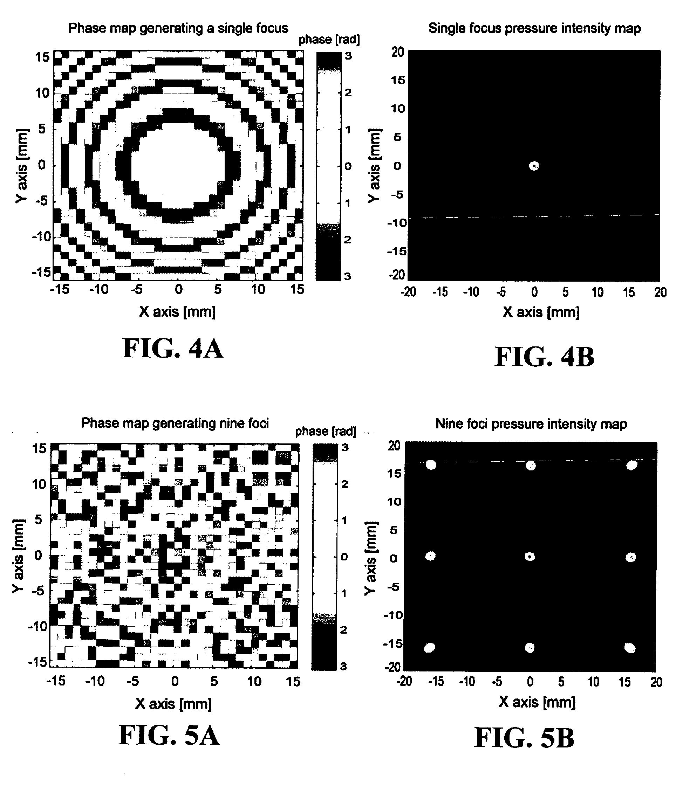

According to some embodiments of the present invention, the acoustic energy transmission pattern defines relative phases of the transmissions of the acoustic energy elements so that they are varied in such a way that the effective transmission pattern of the array is reinforced in a desired direction and suppressed in undesired directions. For example, FIG. 4A which depicts a phase map of a transmission pattern for producing a single focus in a certain target area, placed 60mm away from a transducer along the transducer's central axis, while FIG. 4B depicts the calculated acoustic intensity at the target area. Another example is described in FIGs. 5A and 5B which depict a phase map of a transmission pattern calculated using GSW algorithm for nine foci, for example similarly to the described below, and a resultant acoustic intensity at the target area respectively. Optionally, the acoustic energy transmission pattern defines interludes between the transmissions.

The following set of equation allows calculating a transmission pattern that defines the transmission characteristics of each one of a plurality of acoustic energy elements for applying target acoustic pressures on a plurality of ROIs.

For example, pressure at the target site or point $r$ is calculated to the particle velocity normal to the source's surface by the Rayleigh-Sommerfeld integral over the source's surface:

Equation 1:
$$p(r) = \frac{j\rho ck}{2\pi} \int u(r') \frac{e^{-jkd_{rr'}}}{d_{rr'}} dS$$

where $\rho$ denotes the medium's density, c denotes a velocity of sound in the medium, $k$ denotes the number of waves where $u(r') = |u(r')|e^{j\varphi r'}$ denotes a complex velocity at point $r'$ on the source's surface and $d_{rr'}$ denotes a distance between $r$ and $r'$.

To compute the transmission pattern created by a multi focused acoustic wave source, such as a phased array with N acoustic energy elements, such as US transducers, for forming the target acoustic pressures, one or more simplifying assumptions are made. Optionally, the assumption is that each acoustic energy element of the phased array produces a spherical wavefront emanating from a point at the center of the acoustic energy elements, with amplitude proportional to the elements' area $S_{el}$. Collecting constants into a single constant $K = \dfrac{\rho ck}{2\pi} S_{el}$ , the pressure at $r_m$ which denotes the location of the m$^{th}$ target sites (m=1,..., M) becomes:

Equation 2:
$$p(r_m) = jK \sum_n d_{mn}^{-1} |u_n| e^{j\varphi_n} e^{-jkd_{mn}}$$

where $d_{mn}$ denotes a distance between the center of the n$^{th}$ element and the m$^{th}$ target. This equation may also be written in matrix notation as follows:

Equation 3:
$$\underline{p} = H\underline{u}$$

where the relation between the excitation of the n$^{th}$ acoustic energy element and the pressure at the m$^{th}$ target is given by $H(m,n) = jKd_{mn}^{-1}e^{-jkd_{mn}}$ ; where $u$ denotes the Nx1 excitation vector $u_n = |u_n|e^{j\varphi n}$ and $p$ denotes the Mx1 vector describing the pressure's complex amplitude at each target. The ultrasonic intensity at the target is given by $I_m = \dfrac{|p(r_m)|^2}{2\rho c}$ . A single, high intensity focus is obtained by setting each element's phase to $\varphi_n = kd_{n1}$ and all element's amplitudes to the maximum $U = |u|_{max}$, which results in $p(r_1) = jKU \sum_n d_{1n}^{-1}$ and $I_1 = \dfrac{\left(KU \sum_n d_{1n}^{-1}\right)^2}{2\rho c}$ which is the maximum intensity deliverable to a single point at the same distance from the array.

Optionally, the Fresnel's approximation of Equation 2, is used where the distance between two points is simplified, for example see Goodman, J.W., Fourier Optics. 3 ed. 2005, Englewood, Colorado: Roberts & Company Publishers, which is incorporated herein by reference.

The expression for the distance is

$$d_{mn} = \sqrt{(x_m - x_n)^2 + (y_m - y_n)^2 + z^2} = z\sqrt{1 + \frac{(x_m - x_n)^2}{z^2} + \frac{(y_m - y_n)^2}{z^2}}$$ and after a first order binomial expansion $d_{mn} \approx z\left[1 + \frac{1}{2}\left(\frac{x_m - x_n}{z}\right)^2 + \frac{1}{2}\left(\frac{y_m - y_n}{z}\right)^2\right]$ This expression replaces $d_{mn}$ in the phase term of equation 2, while the $0^{th}$ order expansion - $z$ - is used for the amplitude term, so the approximated equation is as follows:

Equation 4:
$$p(r_m) \approx jKz^{-1}e^{-jkz}\sum_n |u_n| e^{j\varphi_n} e^{-j\frac{k}{2z^2}\left[(x_m - x_n)^2 + (y_m - y_n)^2\right]}$$

According to the present invention, the multi focused acoustic wave source is controlled using a phase-only control that uses phase only weighting of phased array element excitations. For a phase-only phased array, the excitation amplitude is constant, so that the terms summed in Equation 4 are all phase terms, which is important for the derivation of several computer generated holography (CGH) algorithms. This approximation is valid if the targets are in the array's intermediate near-field or far field, for example if the axial distance z between the array and target planes is greater than

$\frac{D^2}{4\lambda}$, where D denotes some characteristic diameter of a transducer, see Angelsen, B.A.J., Ultrasound imaging: waves, signals and signal processing. Vol. 1. 2000, Trondhejm: Emantec. As such an assumption may require a target distance that is too large for a practical setup a limited approximation may be performed. The limited approximation approximates

$d_{mn}^{-1} \approx z^{-1}$ in the amplitude expression. This approximation is valid at shorter distances yet allows deriving a modified version of the algorithms as the terms summed in Equation 4 are again only phase terms.

Now, as shown at 204, the multi focused acoustic wave source is operated according to the transmission pattern so as to apply the target acoustic pressures on the ROIs.

As described above, the transmission pattern may be a spatiotemporal pattern. Such a pattern may be adjusted for reducing speckles, for example as described in U.S. Patent Application No. 12/691,083, filed on January 21, 2010. In use the computing unit 103 may compute a speckle reduction adjustment for the transmission pattern and the controller 105 may operate the multi focused acoustic wave source according to the speckle reduction adjustment.

Reference is now made to an exemplary calculation of a field created by an acoustic wave source, such as a phase-only phased array with uniform velocity amplitudes, using Equation 2. The exemplary calculation is based on the geometry and physical properties of a virtual planar phased array that operates with a central frequency of 2.3 MHz, has N=1024 acoustic energy elements arranged over an aperture of 32x32 mm where each acoustic energy element has an area of 1mm$^2$, as shown in FIG. 4A that depicts the structure and element phases of an exemplary ultrasound phased array. The calculation of the resultant field intensity according to Equation 2, at a plane parallel to the phased array plane and 60 mm distant from it appears in FIG. 4B. FIG. 5A depicts the phases calculated using the GSW algorithm (explanation below) for optimal generation of a field with 9 focal spots, while FIG. 5B depicts the resultant field intensity at the same 60 mm distant plane. In these examples, the field maps have a pixel area of $S = \frac{\lambda}{4} \times \frac{\lambda}{4} = (0.16)^2 mm^2$

Reference is now made to a description of a number of processes of computing the array phases for generating an intensity distribution, optionally optimal. The processes are based on a pseudo-inverse (PINV) algorithm, a random mask (RM) algorithm, a random superposition (SR) algorithm, a Gerchberg-Saxton (GS) algorithm and/or weighted Gerchberg-Saxton (GSW) algorithm, see Di Leonardo, R., F. Ianni, and G. Ruocco, Computer generation of optimal holograms for optical trap arrays. Opt. Express, 2007. 15(4): p. 1913-1922, and Ebbini, E.S. and C.A. Cain, Multiple-focus ultrasound phased-array pattern synthesis: optimal driving-signal distributions for hyperthermia. IEEE Transactions on Ultrasonics, Ferroelectrics and Frequency Control, 1989. 36(5): p. 540-548, which are incorporated herein by reference.

The performance of each process in creating a pattern may be quantified using the following equation, which may be referred to herein as A-normalized efficiency:

$$e = \frac{1}{P_s} \sum_m P_m$$

Equation 5:

where $P_m \equiv S \cdot \sum_{l=1}^{L} I_{m,l}$ denotes an estimation of power delivered to the mth target computed with pixels having an area S, for example as defined above, and $P_s$ denotes power delivered to a pattern having a single focus which is located at the center of the target plane.

The uniformity of power delivery to a target may be described as follows:

$$u = 1 - \frac{P_{max} - P_{min}}{P_{max} + P_{min}}$$

Equation 6:

where $P_{max} = \max_m \{P_m\} = \max_m \left\{ S \cdot \sum_{l=1}^{L} I_{m,l} \right\}$ and $P_{min}$ is defined in a similar fashion.

According to some embodiments of the present invention, the phase calculation process is based on a PINV algorithm. First, a minimum norm solution of the matrix Equation 3 is computed using a pseudo-inverse of H where $\underline{u} = H^H (HH)^{-1} p$ and $H^H$ denotes the conjugate transpose of $H$. The pressure applied by the generated acoustic energy field is as set in $\underline{p}$ at the chosen points in space, for example the ROIs, allowing good uniformity, and that $\|\underline{u}\|$ is minimal, not necessarily a beneficial feature in terms of efficiency. Therefore, the following iterations are aimed at obtaining a solution with a more uniform excitation vector by introducing the weighting matrix $W$ into the equation: $\underline{u} = WH^H (HWH^H)^{-1} \underline{p}$. $W$ is initialized as $W^0 = I$ and its diagonal entries are updated as to comply with the following: $W'_{nm} = \left| u_n^{t-1} \right|^{-1}$. When the amplitudes reach agreeable uniformity at some step $t = \tau$, the phases of the result are used to drive the phased array:

$$\varphi_n^{PINV} = \arg \left\{ \left[ W^\tau H^H \left( HW^\tau H^H \right)^{-1} \underline{p} \right]_n \right\}$$

Equation 7:

Multifocal patterns with uniform power distribution to each of the foci may define a required pressure vector as $p_m \equiv 1$.

According to some embodiments of the present invention, the phase calculation process is based on an RM algorithm. In such a process, each one of the m targets is randomly selected for each of the $n$ acoustic energy elements where $\varphi_n = kd_{nm}$ is set. In this embodiment, each element processes a single target as if it was the only focal point.

According to some embodiments of the present invention, the phase calculation process is based on an SR algorithm. In this embodiment, the algorithm maximizes the sum of real parts in the complex amplitude $\sum_m \mathrm{Re}\left\{ \tilde{p}_m e^{-j\theta_m} \right\}$, in which $\tilde{p}_m = \sum d_{mn}^{-1} \exp\left[ j\left( \varphi_n - kd_{mn} \right) \right]$ simply the pressure $p_m$ defined in equation 2, except for disregarded constants, and $\theta_m$ are random variables distributed uniformly in $[0, 2\pi]$. Optionally, the pressure amplitudes projected on random directions in the complex plane are maximized by requiring the following $\frac{\partial}{\partial \varphi_n} \sum_m \mathrm{Re}\left\{ \tilde{p}_m e^{-j\theta_m} \right\} = 0$ and by obtaining the following:

$$\varphi_n^{SR} = \arg \left\{ \sum_m d_{nm}^{-1} e^{j\left(kd_{nm} + \theta_m\right)} \right\}$$

Equation 8:

According to some embodiments of the present invention, the phase calculation process is based on a GS algorithm. In such a process, a limited approximation is used to simplify the sum of pressure magnitudes delivered to the targets:

$$\text{Equation 9:} \quad \sum_m \left| \tilde{p}_m \right| \approx z^{-1} \sum_m \left| \sum_n \exp\left[ j\left( \varphi_n - kd_{mn} \right) \right] \right|$$

Now the distance $z$ in the simplified expression for $\tilde{p}_m$ is uniform and may be neglected as well in the optimization process, which results in the solution

$$\text{Equation 10:} \quad \varphi_n^{GS} = \arg\left\{ \sum_m \exp\left( jkd_{nm} \right) \frac{\tilde{p}_m}{\left| \tilde{p}_m \right|} \right\}$$

As $\tilde{p}_m$ is not known this is an implicit solution and is optionally used as an iteration formula, in which the phases are initially determined by a different algorithm (e.g. RM, SR or any other algorithm), and at each iteration step the phases are computed according to the pressure field resulting from the previous step's phases:

$$\text{Equation 11:} \quad \varphi_n^{GS}\left( t \right) = \arg\left\{ \sum_m e^{jkd_{nm}} \frac{\tilde{p}_m^{t-1}}{\left| \tilde{p}_m^{t-1} \right|} \right\}$$

According to some embodiments of the present invention, the phase calculation process is based on a GSW algorithm, which is an extension of the GS algorithm. This algorithm maximizes a similar weighted sum of magnitudes $\sum_m w_m \left| \tilde{p}_m \right|$; but under the constraint that all the target amplitudes are identical. This results in a weighted iteration formula:

$$\text{Equation 12:} \quad \varphi_n^{GSW}\left( t \right) = \arg\left\{ \sum_m w_m^t \exp\left( jkd_{nm} \right) \frac{\tilde{p}_m^{t-1}}{\left| \tilde{p}_m^{t-1} \right|} \right\}$$

in which the weights are initialized as $w_m^0 \equiv 1$ and iterated $w_m^t \equiv w_m^{t-1} \frac{\left\langle \left| \tilde{p}^{t-1} \right| \right\rangle}{\left| \tilde{p}^{t-1} \right|}$.

Optionally, a non-uniform field is calculated using the above CGH algorithms by weighting each target m in the relevant sum by an appropriate constant. For example, a non uniform field may be calculated using Equation 11, modified as follows to:

$$\text{Equation 13:} \quad \varphi_n^{GSW}\left( t \right) = \arg\left\{ \sum_m a_m w_m^t e^{jkd_{nm}} \frac{\tilde{p}_m^{t-1}}{\left| \tilde{p}_m^{t-1} \right|} \right\}$$

For example, the selection of $a_k = 2a_l$ results with pressure amplitude at the $k^{th}$ target which is twice the amplitude at the $1^{th}$ target.

The CGH algorithms may also be implemented using the angular spectrum for computing the forward and backward field propagations. In such an embodiment, the projected fields are calculated with improved accuracy utilizing fast Fourier Transforms (FFT), which may be advantageous when generating continuous patterns. Examples of such calculations are described in Clement, G. T. and K. Hynynen (2000, "Field characterization of therapeutic ultrasound phased arrays through forward and backward planar projection", The Journal of the Acoustical Society of America 108(1): 441-446). Iterative updating of the phases, where it is required, is performed according to the principles described above, for each specific algorithm.

According to some embodiments of the present invention, the system of patterning a multi-focal acoustic energy which is described above may be used for simultaneously changing the volume of an intra-bilayer membrane space in a plurality of bilayer membranous structures, such as cells. The intra-bilayer membrane space may be of cellular membranes of one or more bilayer membranous structures of a target biological tissue, artificial membranes of bilayer membranous structures, organelles, for example the nucleus, mitochondria, and/or endoplasmic reticulum, microbes, microorganisms, and/or liposomes. In such embodiments, the system may be used for generating desired bioeffects in a target biological tissue, for example creating pores or ruptures in the bilayer membranous structures bilayer membranes for changing a rate of introducing exogenous material into the intra bilayer membranous structure space, such as cellular space (cytoplasm), stimulating and/or inhibiting one or more cellular processes, and/or changing one or more mechanical characteristics of the cells. The system may be used for releasing content of membranous delivery vessels having a bilayer membrane, for example for releasing medicaments at a desired venue and/or timing in the body. Such a release mechanism may be generated by transmitting an acoustic energy in a spatiotemporal pattern having various amplitudes, frequencies and/or phases which is set to create pores and/or ruptures in the bilayer membrane of the vessels. Such a system may be used to impalement the methods described in U.S. Patent Application No. 61/331,451, filed on May 5, 2010. Optionally, the system is used to apply acoustic waves in a spatiotemporal pattern having an adjusted effect on the membranous structure space.

It is expected that during the life of a patent maturing from this application many relevant methods and systems will be developed and the scope of the term multi focused acoustic wave source, a computing unit, a controller, and a measuring unit is intended to include all such new technologies *a priori.*

As used herein the term "about" refers to $\pm$ 10 %.

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

## EXAMPLES

Reference is now made to the following examples, which together with the above descriptions illustrate some embodiments of the invention in a non limiting fashion.

The experiments utilizing the a method that is based on computing forward and backward projections based on Equation 4, were performed using an ultrasound phased array transducer of a magnetic resonance guided FUS system of Insightec™ and/or a 1.5T magnetic resonance imaging (MRI) system of general electric™ (GE). The ultrasound phased array's arrangement is as described below, except that it is limited to the transmission of 8 phases, which span the $2\pi$ phase dimension. Optionally, the ultrasound phased array transmits acoustic energy to a target tissue via degassed aqueous solution interface.

A thermal rise induced by the acoustic field was measured using a gradient echo MR sequence having an a repetition time to echo time ratio (TR/TE) of 25.2ms /12.4ms, a field of view (FOV) of 20x20cm, and/or a width, which may be referred to as a slice-thickness, of 3mm. This acoustic field having a linear dependency of proton resonance frequency (PRF) of water molecule and temperature. The linear dependency is defined as follows:

Equation 14:
$$\Delta T = \frac{\Delta \varphi}{C \cdot \gamma \cdot B_0 \cdot TE}$$

where $C$ = -0.0091$PPM/°C$ denotes a constant of proportionality, $\gamma$ denotes the proton gyromagnetic ratio, $B_0$ denotes the magnetic field strength, $TE$ denotes echo time and $\Delta\varphi$ denotes phase difference between MR phase images measured before and/or during heating.

The temperature rise at each focal spot was evaluated by taking the maximal $\Delta T$ in a region of 7x7 pixels, which is equivalent to 5.46x5.46 mm, around the targeted focal point. The total temperature elevation is calculated as the sum of the values evaluated for each focal spot, and the pattern uniformity was computed as $u = 1 - \dfrac{\Delta T_{max} - \Delta T_{min}}{\Delta T_{max} + \Delta T_{min}}$,

where $\Delta T_{max} = \max_{m}\{T_m\}$, i.e. the temperature rise in the pixel within the specified targets which exhibited the highest temperature rise, and $\Delta T_{min}$ is defined similarly.

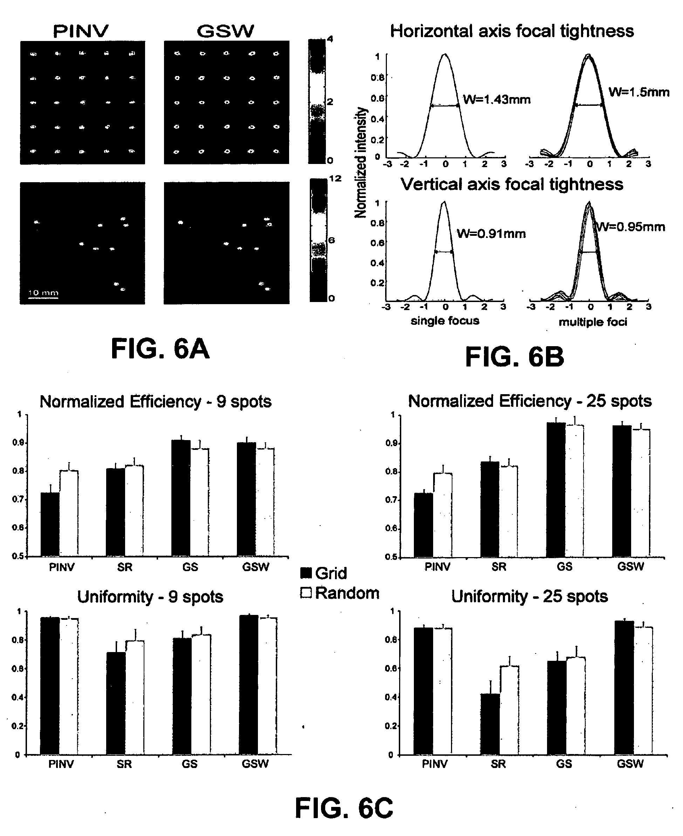

Prior to physical measurements, a simulation study was performed, where the phases required generating multi-focal patterns were evaluated using each of the algorithms mentioned above, in order to estimate their relative performance and computing the resulting efficiency and uniformity. The generated patterns were either symmetric-grid or pseudo-random patterns, situated on a plane parallel to the phased array plane at a distance of $z = 60mm$. A preliminary subset of simulations showed that the algorithms generate multifocal fields with differences in the intensity levels and uniformities between the different foci, for example as shown in FIG. 6C.

The focal spots were found to have similar sizes in each one of the aforementioned algorithms. The focal profiles on horizontal and vertical axes of a nine foci symmetric grid pattern are similar arid only slightly wider than the profile of a single focus, for example as depicted in FIG. 6B. The mean full-width at half maximum (FWHM) of the foci is 0.95 ±0.02 mm mean ± standard deviation (std) on the vertical axis and 1.50mm ± 0.03mm on the horizontal axis, compared with 0.91mm and 1.43mm on the vertical and horizontal axes respectively for a single focal spot. The asymmetry of the foci results from the asymmetry of the phased array aperture that is wider on the vertical axis, leading to a tighter vertical focus.

A quantitative evaluation was based on two sets of multi-focal patterns, with different levels of sparseness. In the first set, nine foci were created within a target square of 32x32 mm$^2$, and in the second set, 25 foci were created within the same square, as shown at FIG. 6A. The sets were composed of 12 pseudo-random patterns and 12 patterns which are versions of an axes-aligned grid rotated at an angle of $\theta = \dfrac{k}{12} \cdot \dfrac{\pi}{2}$, k=0,1,...,11. The power delivered to each focus was quantified by summing the intensity delivered to the FWHM area, approximated by a 1.5mm x 0.75mm rectangular area surrounding the targeted point. In order to avoid overlaps between foci in the pseudo-random set case, the distance between any two target sites is set as at least 1.5 mm.

As shown at FIG. 6C, the SR algorithm yielded patterns which are the least efficient and uniform and the GS algorithm generated patterns which are the most efficient. The GSW algorithm generated patterns which are the most uniform and only about 1-2% less efficient than the GS algorithm.

The patterns of GSW and PINV algorithms show similar uniformities except for the case of the 25 foci grids in which the GSW algorithm yields patterns which have a mean uniformity which is 5% larger. In contrast; GSW algorithm yielded patterns with a mean efficiency that is 17%-23% larger for grid patterns and 8%-15% larger for pseudorandom patterns, a difference which increased as the number of foci grew. Overall, the combination of the highest efficiency and uniformity from all the tested algorithms was achieved by the GSW algorithm.

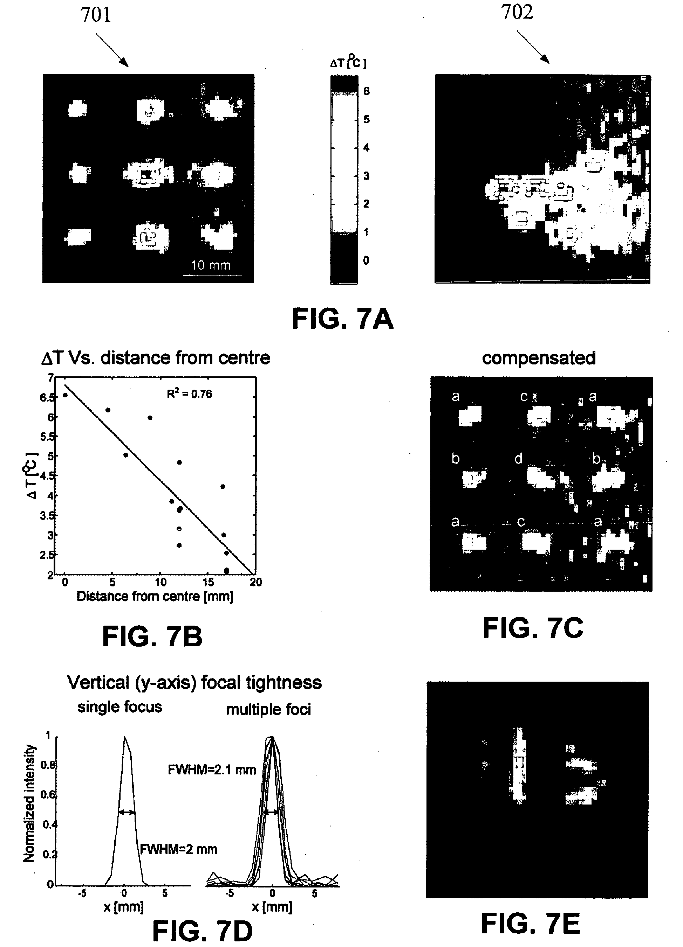

Reference is now made to FIGs. 7A-4E, which depict the multifocal ultrasonic distribution of an acoustic energy field generated by a phased-array with 987 acoustic energy elements according to a pattern calculated by a GSW algorithm, according to some embodiments of the present invention.

As shown at FIG. 7A, acoustic field induces a pattern 701 having nine spots which may be symmetrically arranged in a grid and a pseudorandom pattern 702 generated on a plane parallel to the array plane at a distance of 60 mm. The successful production of a multi-focal field is evident from the image 701. It should be noted that the temperature elevation at each foci has relatively low uniformity, quantified as U=0.48 in the pattern depicted in 701 and U=0.54 in the pattern depicted in 702. This is an outcome of the acoustic energy elements of the array which have a radiation profile which deviates from a spherical profile implied by the assumptions taken during the aforementioned calculations. As such, the focal temperature elevation is dependent on the distance of the focus from a target plane center defined herein as the point in the target plane which intersects with a vector normal to the array plane and situated at the array center. This dependency is shown in FIG. 7B which plots the temperature elevations in the foci of the patterns in FIG. 7A against their distance from the center. The decrease in temperature elevation appears to be less drastic on the y-axis than the x-axis, which may be due to the array's asymmetric aperture.

The non-uniformity may be corrected using Equation 13 to compute the phases required for a non-uniform field, choosing the relative target weights so they compensate for the array's inherent non-uniformity. FIG. 7C shows a compensated 9 spots grid, in which the foci points are marked [a, b, c, d] and weighted by [1, 0.8, 0.74, 0.52] respectively by the weights $a_m$ in equation 13. This results in greatly improved uniformity, quantified as U=0.9, at the expense of an 11% reduction in the total temperature elevation. In this example, the pixel size is 0.78 mm, almost the focal FWHM described above. However, under these limitations some upper bound to the focal size may be evaluated. We investigate the focal size in the compensated grid image which is obtained after 13.2 seconds of irradiation, finding the FWHM along the vertical and horizontal scale to be $2.2 \pm 0.1$ mm and $2.4 \pm 0.4$ mm and respectively (mean $\pm$ std), the former shown in figure 3D. These values are on a scale similar to the simulated values. The FWHM of a single central focus is 2.1 and 2.2 mm on the vertical and horizontal axes respectively, slightly smaller than the FWHM in the multi-focal field, in good accordance with the simulations, as well as the vertical / horizontal asymmetry. FIG. 7D is a graph that depicts the focal tightness as quantified for a single central focus and a multifocal pattern.

FIG. 7E depicts a complex pattern created by 22 focal points. The pattern spells out the word "US", and computes the required phases with the GSW algorithm. The complex pattern depicted in FIG. 7E is a result of temperature elevation after 19.8 seconds of sonication.

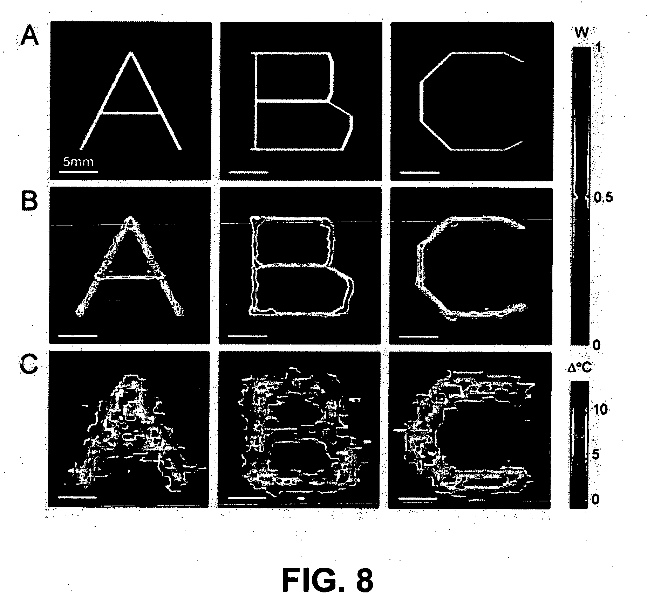

Results of experiments and simulation utilizing a method that uses the angular spectrum for computing the forward and backward projections based on Fast Fourier transform (FFT) calculations are depicted in FIG. 8. The simulations were performed using 2048x2048 pixels matrixes of complex numbers to span an acoustic field plane of 10.2x10.2cm at 50x50$\mu$m resolution. The three letter patterns, 'A', 'B' and 'C', were manually built into 1.5x1.5cm masks (the row in marked with the notationA) to represent the requested target fields at a parallel plane, 25mm from the transducer plane. The simulation results are generally smooth, uniform and show that most of the power that is indeed directed at the required pattern (the row in marked with the notation. B) The experiments were performed and measured using a setup similar to the one described in the previous pages (results are depicted in the row marked with the notation C). MRI temperature elevation images were acquired on a GE 1.5T scanner equipped with Insightec FUS transducer (FSPGR sequence, TR/TE=35.8/22.8ms, FOV=12.8x12.8cm, slice thickness=5mm, in plane resolution of 0.5x0.5mm). A reference scan that was taken before sonication was subtracted from a scan taken 5 seconds after beginning of sonication to measure the temperature elevation. Sonication of 49.4W acoustic power and 10sec duration was performed to a focal plane of 25mm using Insightec™ ultrasound phased array transducer, which was originally designed for the treatment of prostate tumors. The results resemble the simulation results, showing generally smooth temperature elevations. The measured patterns have wider lines than the simulated ones, due to thermal diffusion: the FWHM of a point source diffused heat profile is about 2mm after 7.5sec of diffusion time, the average diffusion time in the thermal acquisition. To examine the applicability of ultrasound (US) for the stimulation of neural cells in the eye, we studied the retina's response to US stimuli. We measured visual evoked potentials (VEPs) from anesthetized rats (SD, weight 230$\pm$26 g mean $\pm$ standard deviation, n=3), in response to light flashes and to ultrasonic pulses. Rats were anesthetized using a ketmaine : xylazine : acepromazine cocktail (induction - 50 : 6.25 : 1.25 mg/Kg body weight; anesthetic maintenance with ketamine : diazepam 50 : 2.5 mg/Kg body weight). The flashes were projected from a bright blue LED (10 ms ON tim e, once every 2 secs) directed to the rat's eye while VEPs were recorded using a pair of needle electrodes (Axon systems, DSN1260, 13 mm 27G monopolar ), one inserted subcutaneously contralaterally to the stimulated eye, near the lambda in a rostro-caudal direction 1-2 mm lateral to the median plane, the other under the contralateral ear, while another subcutaneous electrode on the animal's trunk served as ground. The recorded VEP signal was amplified, filtered at 0.1-500 Hz and digitized by a single device (Psychlab, 4 channels EEG). Additional digital processing included band pass filtering (8.5-42 Hz, -3dB cutoff frequencies) and averaging of the traces triggered by the stimulus onset (at least 150 repeats), performed on Matlab.

Subsequently, a 1MHz US transducer (Imasonic) was coupled to the eye using a custom-built conical Plexiglass coupler filled with degassed water and sealed using a thin hydrophobic membrane (parafilm), and ophthalmic gel (Viscotears). US pulses were generated by a function generator (Tabor 8024), amplified (50 dB, ENI 550L) and fed to the transducer which emitted 10 millisecond (ms) bursts once every 2 seconds ($I_{SPTP}$=10-17 W/cm2, 100 cycles/pulse, pulse repetition frequency: 1667 Hz), while the animal's ears were sealed with dental elastomer to avoid auditory artifacts. Responses were recorded and analyzed in an identical fashion and care was taken to minimize electrode movement during the experiment. By $I_{SPTP}$ we refer to the pulse average intensity $I_{PA}$, measured specifically at the spatial location in which the intensity is maximal.

As a control we eliminated the US-eye coupling by moving the cone a few millimeters away and repeated the stimulation. Additionally, injection of tetrodotoxin (TTX) to the vitreous space was performed by gently piercing the sclera-cornea boundary with a needle (30 G), inserting a micro-liter syringe (Hamilton) and injecting 1 $\mu$l of TTX concentrated at 500 $\mu$M. Assuming a vitreous humor volume of approximately 50 $\mu$l the final average TTX concentration was 10 $\mu$M. After the administration of TTX the US stimulation was repeated.

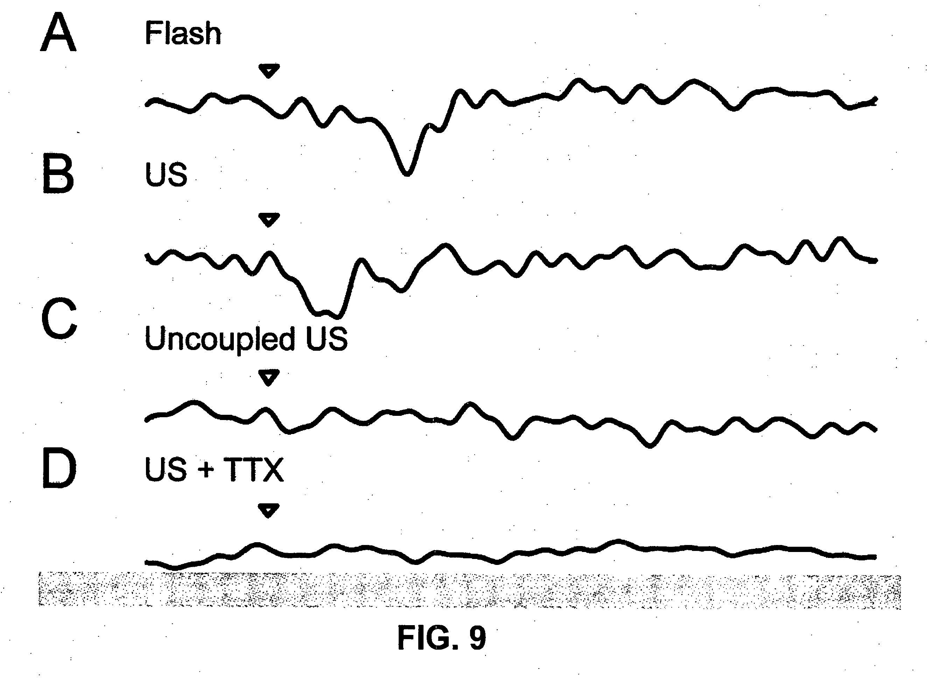

An example of averaged traces from one experiment session shows, as depicted in FIG. 9, evident responses to the flash and US stimuli (see trace marked with the notations A and B respectively) and none in the control condition (see trace marked with the notation C). Similarly, no response was found after administration of TTX (see trace marked with the notation D).

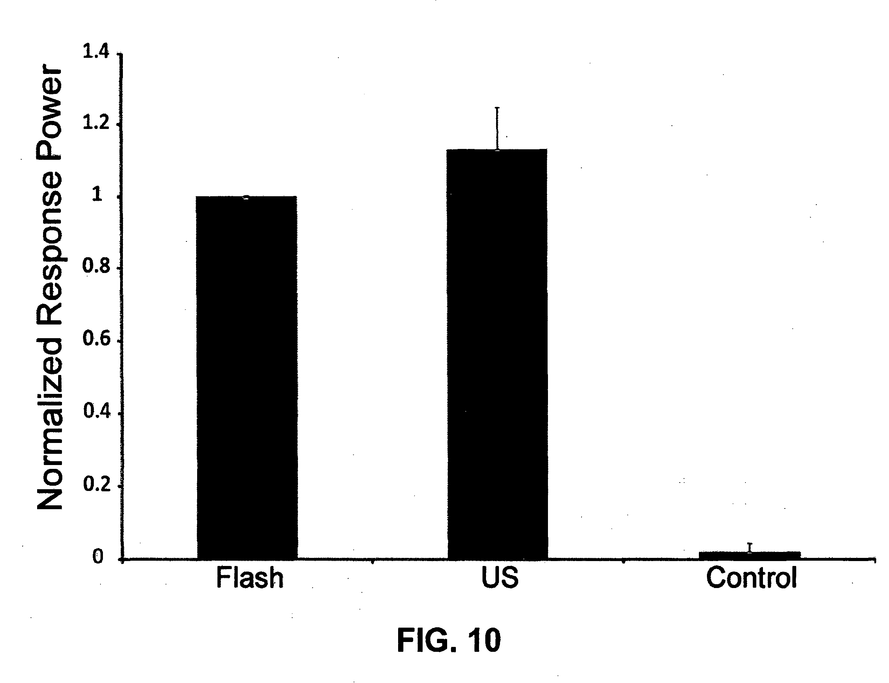

We defined the response power as the difference in power between the power during stimulus response and during baseline, calculated by taking the square of mean subtracted voltage traces, designating the baseline power as the mean power 0.7-0.2 seconds before the stimulus onset and the response as the mean power 0-0.15 seconds after onset. The results grouped from all animals and normalized to the response to flashes, for example as depicted in FIG. 10, show that on average, US stimulation elicited a response power larger by 13% with 12% standard error (SE). In contrast, the mean response power in the uncoupled ultrasound control condition is only 2% $\pm$ 2% (average $\pm$ SE) of the response power to flashes.

**Claims**

1. A system of patterning a multi-focal acoustic energy transmission, comprising:

   an input interface (102) adapted to receive a plurality of target acoustic pressures to be applied on a plurality of focal spots of a target area in at least one cellular tissue; **characterised by**:

   a computing unit (103) adapted to compute, using an computer generated holography algorithm, a multifocal transmission pattern of an acoustic energy source, said multifocal transmission pattern set so as to operate each of a plurality of acoustic energy elements of said acoustic energy source to apply said plurality of target acoustic pressures on a plurality of regions of interests in said target area, said multifocal transmission pattern defining a phase of a plurality of acoustic energy transmissions each for each one of a plurality of acoustic energy elements so that said plurality of acoustic energy transmissions contributes to said plurality of target acoustic pressures which are applied on said plurality of regions of interests; and
   a controller (105) adapted to operate said plurality of acoustic energy elements to transmit said plurality of acoustic energy transmissions on said target area.

2. The system of claim 1, wherein said computing unit is adapted to compute a speckle reduction adjustment for said multifocal transmission pattern, said controller operates said acoustic energy source according to said multifocal transmission pattern in light of said speckle reduction adjustment.

3. The system of any of claim 1-2, wherein said acoustic energy source has a plurality of dynamic acoustic energy elements and said multifocal transmission pattern is a spatiotemporal pattern.

4. The system of any of the previous claims, wherein said acoustic energy source is adapted to transmit said multifocal transmission pattern with energy in frequencies at the range between 1 Mega Hertz (MHz) and 20 MHz.

5. The system of any of the previous claims, wherein said acoustic energy source is adapted to transmit said multifocal transmission pattern with energy having a pulse average acoustic intensity of up to 100W/cm^2.

6. The system of any of the previous claims, wherein said computing unit (103) is adapted to compute said multifocal transmission pattern as a spatiotemporal pattern for applying said plurality of target acoustic pressures each varing over a period each in a different region of interest (ROI).

7. The system of any of the previous claims, wherein said computing unit (103) is adapted to receive instructions for applying said plurality of target acoustic pressures, each in a different region of interest (ROI); wherein said instructions are generatable according to readings of at least one sensor.

8. The system of claim 1, wherein said computing unit (103) is adapted to compute said plurality of phases according to at least one of a random superposition (SR) process, a Gerchberg-Saxton (GS) process, and a weighted Gerchberg-Saxton (GSW) process.

9. The system of any of the previous claims, said computing unit (103) is adapted to compute a transmission spatiotemporal pattern defining a plurality of amplitudes each for another of a plurality of dynamic acoustic energy elements, said controller (105) is adapted to operate said plurality of acoustic energy elements by adjusting said plurality of dynamic acoustic energy elements to transmit according to said plurality of amplitudes.

10. The system of any of the previous claims, wherein said computing unit (103) is adapted to compute a speckle reduction adjustment for said transmission pattern, said controller (105) is adapted to operate said plurality of acoustic energy elements according to said speckle reduction adjustment.

11. The system of any of the previous claims, wherein said computing unit (103) is adapted to compute said multifocal transmission pattern of said acoustic energy source by a plurality of iterations wherein in each said iteration a plurality of phases are computed according to the pressure field resulting from calculations of previous iterations of said plurality of iterations.

12. The system of any of the previous claims, wherein said acoustic energy source is placed on glasses or on hydrogel contact lenses and adapted to stimulate neurons in the retina.

13. The system of any of the previous claims, wherein said system is mountable on a subject.

**Patentansprüche**

1. System zur Strukturierung einer Schallenergieübertragung mit mehreren Fokussen, umfassend:

   eine Eingangsschnittstelle (102), die so ausgelegt ist, dass sie eine Mehrzahl von Ziel-Schalldrücken empfängt, die an eine Mehrzahl von Fokuspunkten eines Zielbereichs in wenigstens einem zellulären Gewebe angelegt werden sollen, gekennzeichnet durch:

   eine Recheneinheit (103), die so ausgelegt ist, dass sie unter Verwendung eines computergenerierten Holographiealgorithmus ein Übertragungsmuster mit mehreren Fokussen einer Schallenergiequelle berechnet, wobei das Übertragungsmuster mit mehreren Fokussen so eingestellt ist, dass jedes einer Mehrzahl von Schallenergieelementen der Schallenergiequelle so betrieben wird, dass sie die Mehrzahl von Ziel-Schalldrücken an eine Mehrzahl relevanter Regionen im Zielbereich anlegt, wobei das Übertragungsmuster mit mehreren Fokussen jeweils eine Phase einer Mehrzahl von Schallenergieübertragungen für jedes einer Mehrzahl von Schallenergieelementen definiert, so dass die Mehrzahl von Schallenergieübertragungen zur Mehrzahl von Ziel-Schalldrücken beiträgt, die an die Mehrzahl relevanter Regionen angelegt werden; und
   eine Steuereinheit (105), die so ausgelegt ist, dass sie die Mehrzahl von Schallenergieelementen so betreibt, dass sie die Mehrzahl von Schallenergieübertragungen zum Zielbereich übertragen.

2. System nach Anspruch 1, wobei die Recheneinheit so ausgelegt ist, dass sie für das Übertragungsmuster mit mehreren Fokussen eine Speckle-Reduzierungsanpassung berechnet, und die Steuereinheit die Schallenergiequelle gemäß dem Übertragungsmuster mit mehreren Fokussen unter Berücksichtigung der Speckle-Reduzierungsanpassung betreibt.

3. System nach einem von Anspruch 1-2, wobei die Schallenergiequelle eine Mehrzahl dynamischer Schallenergieelemente aufweist und das Übertragungsmuster mit mehreren Fokussen ein Raum-Zeit-Muster ist.

4. System nach einem der vorangehenden Ansprüche, wobei die Schallenergiequelle so ausgelegt ist, dass sie das Übertragungsmuster mit mehreren Fokussen mit Energie im Frequenzbereich zwischen 1 Megahertz (MHz) und 20 MHz überträgt.

5. System nach einem der vorangehenden Ansprüche, wobei die Schallenergiequelle so ausgelegt ist, dass sie das Übertragungsmuster mit mehreren Fokussen mit Energie mit einer mittleren Schallintensität des Pulses von bis zu 100 W/cm$^2$ überträgt.

6. System nach einem der vorangehenden Ansprüche, wobei die Recheneinheit (103) so ausgelegt ist, dass sie das Übertragungsmuster mit mehreren Fokussen als Raum-Zeit-Muster für das Anlegen der Mehrzahl von Ziel-Schalldrücken berechnet, die in jeweils einer anderen relevanten Region jeweils über einen Zeitraum variieren.

7. System nach einem der vorangehenden Ansprüche, wobei die Recheneinheit (103) so ausgelegt ist, dass sie Befehle zum Anlegen der Mehrzahl von Ziel-Schalldrücken in jeweils einer anderen relevanten Region empfängt, wobei die Befehle gemäß von Messwerten wenigstens eines Sensors generierbar sind.

8. System nach Anspruch 1, wobei die Recheneinheit (103) so ausgelegt ist, dass sie die Mehrzahl von Phasen gemäß einem Zufallsüberlagerungsverfahren (SR), einem Gerchberg-Saxton-Verfahren (GS) und/oder einem gewichteten Gerchberg-Saxton-Verfahren (GSW) berechnet.

9. System nach einem der vorangehenden Ansprüche, wobei die Recheneinheit (103) so ausgelegt ist, dass sie ein Raum-Zeit-Muster der Übertragung berechnet, das eine Mehrzahl von Amplituden für jeweils ein anderes einer Mehrzahl dynamischer Schallenergieelemente definiert, und die Steuereinheit (105) so ausgelegt ist, dass sie die Mehrzahl von Schallenergieelementen betreibt, indem sie die Mehrzahl dynamischer Schallenergieelemente so anpasst, dass sie gemäß der Mehrzahl von Amplituden übertragen.

10. System nach einem der vorangehenden Ansprüche, wobei die Recheneinheit (103) so ausgelegt ist, dass sie eine Speckle-Reduzierungsanpassung für das Übertragungsmuster berechnet, und die Steuereinheit (105) so angepasst ist, dass sie die Mehrzahl von Schallenergieelementen gemäß der Speckle-Reduzierungsanpassung betreibt.

11. System nach einem der vorangehenden Ansprüche, wobei die Recheneinheit (103) so ausgelegt ist, dass sie das Übertragungsmuster mit mehreren Fokussen der Schallenergiequelle durch eine Mehrzahl von Iterationen berechnet, wobei bei jeder Iteration gemäß dem sich aus Berechnungen vorangehender Iterationen der Mehrzahl von Iterationen ergebenden Druckfeld eine Mehrzahl von Phasen berechnet wird.

12. System nach einem der vorangehenden Ansprüche, wobei die Schallenergiequelle auf einer Brille oder auf Hydrogel für Kontaktlinsen angeordnet und so ausgelegt ist, dass sie Neuronen in der Netzhaut stimuliert.

13. System nach einem der vorangehenden Ansprüche, wobei das System an einem Patienten anbringbar ist.

## Revendications

1. Système destiné à établir le diagramme d'une transmission d'énergie acoustique à plusieurs foyers, comprenant :

une interface d'entrée (102) adaptée pour recevoir une pluralité de pressions acoustiques cibles devant être appliquées sur une pluralité de points focaux d'une zone cible dans au moins un tissu cellulaire, **caractérisé par** une unité de calcul (103) adaptée pour calculer, à l'aide d'un algorithme d'holographie généré par ordinateur, un diagramme de transmission multifocale d'une source d'énergie acoustique, ledit diagramme de transmission multifocale étant réglé de façon à faire fonctionner chacun d'une pluralité d'éléments d'énergie acoustique de ladite source d'énergie acoustique pour appliquer ladite pluralité de pressions acoustiques cibles sur une pluralité de régions d'intérêt dans ladite zone cible, ledit diagramme de transmission multifocale définissant une phase d'une pluralité de transmissions d'énergie acoustique chacune pour chacun d'une pluralité d'éléments d'énergie acoustique de telle sorte que ladite pluralité de transmissions d'énergie acoustique contribue à ladite pluralité de pressions acoustiques cibles qui sont appliquées sur ladite pluralité de régions d'intérêt ; et un régulateur (105) adapté pour faire fonctionner ladite pluralité d'éléments d'énergie acoustique pour transmettre ladite pluralité de transmissions d'énergie acoustique sur ladite zone cible.

2. Système selon la revendication 1, dans lequel ladite unité de calcul est adaptée pour calculer un ajustement de la réduction du chatoiement pour ledit diagramme de transmission multifocale, ledit régulateur fait fonctionner ladite source d'énergie acoustique en fonction dudit diagramme de transmission multifocale à la lumière dudit ajustement de la réduction du chatoiement.

3. Système selon l'une quelconque des revendications 1-2, dans lequel ladite source d'énergie acoustique a une pluralité d'éléments d'énergie acoustique dynamique et ledit diagramme de transmission multifocale est un diagramme spatiotemporel.

4. Système selon l'une quelconque des revendications précédentes, dans lequel ladite source d'énergie acoustique est adaptée pour transmettre ledit diagramme de transmission multifocale avec une énergie en fréquences dans la plage entre 1 mégahertz (MHz) et 20 MHz.

5. Système selon l'une quelconque des revendications précédentes, dans lequel ladite source d'énergie acoustique est adaptée pour transmettre ledit diagramme de transmission multifocale avec une énergie ayant une intensité acoustique moyenne d'impulsions jusqu'à 100 W/cm^2.

6. Système selon l'une quelconque des revendications précédentes, dans lequel ladite unité de calcul (103) est adaptée pour calculer ledit diagramme de transmission multifocale en tant que diagramme spatiotemporel pour appliquer ladite pluralité de pressions acoustiques cibles qui varient chacune pendant une période à chaque fois dans une région d'intérêt (RDI) différente.

7. Système selon l'une quelconque des revendications précédentes, dans lequel ladite unité de calcul (103) est adaptée pour recevoir des instructions pour appliquer ladite pluralité de pressions acoustiques cibles, chacune dans une région d'intérêt (RDI) différente ; dans lequel lesdites instructions peuvent être générées selon des valeurs de lecture d'au moins un capteur.

8. Système selon la revendication 1, dans lequel ladite unité de calcul (103) est adaptée pour calculer ladite pluralité de phases selon au moins un processus parmi un processus de superposition aléatoire (SA), un processus Gerchberg-Saxton (GS) et un processus Gerchberg-Saxton pondéré (GSP).

9. Système selon l'une quelconque des revendications précédentes, ladite unité de calcul (103) étant adaptée pour calculer un diagramme spatiotemporel de transmission définissant une pluralité d'amplitudes chacune pour un autre d'une pluralité d'éléments d'énergie acoustique dynamique, ledit régulateur (105) étant adapté pour faire fonctionner ladite pluralité d'éléments d'énergie acoustique en ajustant ladite pluralité d'éléments d'énergie acoustique dynamique pour effectuer la transmission selon ladite pluralité d'amplitudes.

10. Système selon l'une quelconque des revendications précédentes, dans lequel ladite unité de calcul (103) est adaptée pour calculer un ajustement de la réduction du chatoiement pour ledit diagramme de transmission, ledit régulateur (105) étant adapté pour faire fonctionner ladite pluralité d'éléments d'énergie acoustique selon ledit ajustement de la réduction du chatoiement.

11. Système selon l'une quelconque des revendications précédentes, dans lequel ladite unité de calcul (103) est adaptée pour calculer ledit diagramme de transmission multifocale de ladite source d'énergie acoustique par une pluralité d'itérations dans lequel, dans chaque dite itération, une pluralité de phases sont calculées selon le champ de pression résultant de calculs d'itérations précédentes de ladite pluralité d'itérations.

12. Système selon l'une quelconque des revendications précédentes, dans lequel ladite source d'énergie acoustique est placée sur des verres ou de l'hydrogel pour lentilles de contact et adaptée pour stimuler des neurones dans la rétine.

13. Système selon l'une quelconque des revendications précédentes, dans lequel ledit système peut être monté sur un sujet.

**FIG. 1**

EP 2 571 574 B1

FIG. 2A

EP 2 571 574 B1

**FIG. 2B**

EP 2 571 574 B1

FIG. 2C

Providing a multi focused acoustic
waves source | 201

Providing target pressures | 202

Computing a transmission pattern | 203

Operating the source according to the
transmission pattern | 204

FIG. 3

Phase map generating a single focus
phase [rad]

**FIG. 4A**

Single focus pressure intensity map

**FIG. 4B**

Phase map generating nine foci
phase [rad]

**FIG. 5A**

Nine foci pressure intensity map

**FIG. 5B**

FIG. 6A

FIG. 6B

FIG. 6C

FIG. 7A

ΔT Vs. distance from centre

FIG. 7B

compensated

FIG. 7C

Vertical (y-axis) focal tightness

single focus          multiple foci

FIG. 7D

FIG. 7E

**FIG. 8**

A Flash

B US

C Uncoupled US

D US + TTX

FIG. 9

EP 2 571 574 B1

**FIG. 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 69108310 A

- US 61331451 A

### Non-patent literature cited in the description

- **FRY, F.J. ; H.W. ADES ; W.J. FRY.** Production of Reversible Changes in the Central Nervous System by Ultrasound. *Science,* 1958, vol. 127 (3289), 83-84
- **GAVRILOV, L.R ; E.M. TSIRULNIKOV ; I.A.I. DAVIES.** Application of focused ultrasound for the stimulation of neural structures. *Ultrasound in medicine & biology,* 1996, vol. 22 (2), 179-192
- **TYLER, W.J. et al.** Remote Excitation of Neuronal Circuits Using Low-Intensity, Low-Frequency Ultrasound. *PLoS ONE,* 2008, vol. 3 (10), e3511
- **YUSUF TUFAIL ; ALEXEI MATYUSHOV ; NATHAN BALDWIN ; MONICA L. TAUCHMANN ; JOSEPH GEORGES ; ANNA YOSHIHIRO ; STEPHEN I. ; HELMS TILLERY ; WILLIAM J. TYLER.** Transcranial Pulsed Ultrasound Stimulates Intact Brain Circuits. *Neuron,* 2010, vol. 66 (5), 681-694
- **YOO, S.-S. ; A. BYSTRITSKY ; J.-H. LEE ; Y. ZHANG ; K. FISCHER ; B.-K. MIN ; N. J. MCDANNOLD ; A. PASCUAL-LEONE ; F. A. JOLESZ.** Focused ultrasound modulates region-specific brain activity. *Neuro Image,* 2011
- **KRASOVITSKI, B. ; V. FRENKEL ; S. SHOHAM ; E. KIMMEL.** Intramembrane cavitation as a unifying mechanism for ultrasound-induced bioeffects. *Proceedings of the National Academy of Sciences,* 2011
- **COLUCCI, V. et al.** Focused Ultrasound Effects on Nerve Action Potential in vitro. *Ultrasound in Medicine & Biology,* 2009, vol. 35 (10), 1737-1747
- **YUSUF TUFAIL ; ALEXEI MATYUSHOV ; NATHAN BALDWIN ; MONICA L. TAUCHMANN ; JOSEPH GEORGES ; ANNA YOSHIHIRO ; STEPHEN I. HELMS TILLERY ; WILLIAM J. TYLER.** Transcranial Pulsed Ultrasound Stimulates Intact Brain Circuits. *Neuron,* 2010, vol. 66 (5), 681-694

- **YOO, S.-S. ; A. BYSTRITSKY ; J.-H. LEE ; Y. ZHANG ; K. FISCHER ; B.-K. MIN ; N. J. MCDANNOLD ; A. PASCUAL-LEONE ; F. A. JOLESZ.** Focused ultrasound modulates region-specific brain activity. *NeuroImage,* 2011
- **MURATORE, R. ; J. LAMANNA ; E. SZULMAN ; M.S.A. KALISZ ; M. LAMPRECHT ; M.S.M. SIMON ; M.S.Z. YU ; N. XU ; B. MORRISON.** Bioeffective Ultrasound at Very Low Doses: Reversible Manipulation of Neuronal Cell Morphology and Function in Vitro. *8TH INTERNATIONAL SYMPOSIUM ON THERAPEUTIC ULTRASOUND,* 2009
- **DI LEONARDO R ; IANNI F ; RUOCCO G.** Computer generation of optimal holograms for optical trap arrays. *Opt. Exp.,* 2007, vol. 15, 1913-22
- **S. H. WONG ; M. KUPNIK ; R. D. WATKINS ; K. BUTTS-PAULY ; B. T. KHURI-YAKUB.** Capacitive micromachined ultrasonic transducers for therapeutic ultrasound applications. *IEEE Tran. Biomed. Eng.,* January 2010, vol. 57 (1), 114-123
- **K. K. PARK ; H. LEE ; M. KUPNIK ; B. T. KHURI-YAKUB.** Fabrication of capacitive micromachined ultrasonic transducers via local oxidation and direct wafer bonding. *Microelectromechanical Systems, Journal,* February 2011, vol. 20 (2), 95-103
- **GOODMAN, J.W.** Englewood, Colorado. Roberts & Company Publishers, 2005
- **ANGELSEN, B.A.J.** *Ultrasound imaging: waves, signals and signal processing,* 2000, vol. 1
- **DI LEONARDO, R. ; F. IANNI ; G. RUOCCO.** Computer generation of optimal holograms for optical trap arrays. *Opt. Express,* 2007, vol. 15 (4), 1913-1922
- **EBBINI, E.S. ; C.A. CAIN.** Multiple-focus ultrasound phased-array pattern synthesis: optimal driving-signal distributions for hyperthermia. *IEEE Transactions on Ultrasonics, Ferroelectrics and Frequency Control,* 1989, vol. 36 (5), 540-548
- **CLEMENT, G. T. ; K. HYNYNEN.** Field characterization of therapeutic ultrasound phased arrays through forward and backward planar projection. *The Journal of the Acoustical Society of America,* 2000, vol. 108 (1), 441-446